# EUROPEAN PATENT APPLICATION

(11) **EP 4 804 208 A1**
(43) Date of publication of application: **09.09.2026**
(21) Application number: 24904284.7
(22) Date of filing: 13.12.2024
(51) Int. Cl.: G16H 50/50, G16H 10/60, A61B 5/318, G16H 50/20, G16H 50/70, A61B 5/346, G06N 3/0464, G06N 3/0455

(54) **METHOD, DEVICE, AND COMPUTER PROGRAM FOR OBTAINING NEURAL NETWORK MODEL FOR PREDICTING DISEASE ON BASIS OF ELECTROCARDIOGRAM DATA**

(30) Priority: 13.12.2023 KR 20230180363; 03.06.2024 KR 20240072203
(71) Applicant: Medical AI Co., Ltd., Seoul 06180 (KR)
(72) Inventor: KWON, Joonmyoung, Seoul 06180 (KR); HONG, Donggyun, Seoul 06180 (KR); SONG, Junho, Seoul 06180 (KR); JANG, Jonghwan, Seoul 06180 (KR)
(74) Representative: Patentanwaltskanzlei Matschnig & Forsthuber OG
(86) International application number: PCT/KR2024/020323
(87) International publication number: WO 2025/127719

(57) **Abstract**

The present disclosure provides a method, a device, and a computer program for obtaining a neural network model for predicting a disease based on ECG data. According to an embodiment of the present disclosure, a method for obtaining a neural network model for predicting a disease based on time-series data, performed by a computing device comprising at least one processor, comprises: obtaining a plurality of pieces of first divided data by dividing first time-series data; obtaining first training data corresponding to the first time-series data by masking at least one of the obtained plurality of pieces of first divided data; and training a first neural network model through self-supervised learning based on the first training data.

## Description

### TECHNICAL FIELD

The present disclosure relates to artificial intelligence (hereinafter "AI") technology in the medical field, and more specifically, to a method, a device, and a computer program for training a neural network model to predict a disease.

### BACKGROUND OF THE INVENTION

With the recent development of information and communication technology and AI technology, the analysis and utilization of various medical data have become possible. In particular, an electrocardiogram (hereinafter "ECG") is an important biosignal that records the electrical activity of the heart and is essentially used for the diagnosis and monitoring of heart diseases. The analysis of ECG data plays a crucial role in determining the type of heart disease. However, conventional analysis methods have had to rely on a limited workforce, such as professional doctors and researchers, and accordingly, there have been limitations in efficiency and speed.

As methods for analyzing ECG data using AI technology have emerged, the automation of heart disease prediction has been attempted, but there are still unresolved problems. ECG data requires data that reflects various conditions and situations because the signal patterns vary greatly depending on a patient's physiological characteristics, health status, disease history, and the like. However, it is difficult to secure such data sufficiently. High-quality ECG data can only be collected through specialized medical environments and equipment, and due to this, a great deal of time and cost is consumed in data collection. Furthermore, a lack of labeled data may cause a decline in the performance of an AI model, and if the diversity of data is not secured, the generalization performance of the model is also difficult to guarantee.

Although various approaches have been attempted to solve these problems, an appropriate method that can effectively learn the latent features of ECG signals and accurately predict diseases by utilizing limited data has not yet been sufficiently presented.

### SUMMARY OF THE INVENTION

### TECHNICAL PROBLEM

The present disclosure is contrived in response to the aforementioned background, and an object thereof is to provide a method, a device, and a computer program for obtaining a neural network model for predicting a disease based on ECG data.

However, the problems to be solved by the present disclosure are not limited to the aforementioned problems, and other unmentioned problems will be clearly understood from the following description.

### TECHNICAL SOLUTION

To achieve the aforementioned objects, a method for obtaining a neural network model for predicting a disease based on time-series data, performed by a computing device comprising at least one processor, comprises: obtaining a plurality of pieces of first divided data by dividing first time-series data; obtaining first training data corresponding to the first time-series data by masking at least one of the obtained plurality of pieces of first divided data; and training a first neural network model through self-supervised learning based on the first training data.

Alternatively, the training of the first neural network model may comprise: training the first neural network model through generative learning and contrastive learning based on the first training data.

Alternatively, the training of the first neural network model through the generative learning and the contrastive learning may comprise: training the first neural network model through the generative learning based on the first time-series data and output data, the output data being obtained by inputting the first training data to the first neural network model so that the masked divided data of the first training data is reconstructed.

Alternatively, the method may comprise: obtaining a plurality of pieces of second divided data by dividing second time-series data corresponding to a positive pair or a negative pair with respect to the first time-series data; and obtaining second training data corresponding to the second time-series data by masking at least one of the obtained plurality of pieces of second divided data. The training of the first neural network model through the generative learning and the contrastive learning may comprise: training the first neural network model through the contrastive learning using the first training data and the second training data as an input pair.

Alternatively, the method may comprise: extracting an encoder included in the first neural network model when the training of the first neural network model through the generative learning and the contrastive learning is completed; obtaining a second neural network model by combining the extracted encoder and a classifier; and fine-tuning the second neural network model to predict a disease of a patient, through supervised learning based on third time-series data to which preset classes are assigned.

Alternatively, the obtaining of the plurality of pieces of second divided data may comprise: setting the second time-series data to correspond to a positive pair when the first time-series data and the second time-series data are obtained from a single subject, and to a negative pair when the first time-series data and the second time-series data are obtained from different subjects.

Alternatively, the obtaining of the plurality of pieces of second divided data may comprise: setting augmented data corresponding to the first time-series data to correspond to a positive pair with respect to the first time-series data, the augmented data being obtained by adjusting the first time-series data.

Alternatively, the training of the first neural network model through the generative learning and the contrastive learning may comprise: applying, to the first neural network model, a combination of a first loss function corresponding to the generative learning and a second loss function corresponding to the contrastive learning.

Alternatively, the first neural network model may comprise: an encoder configured to extract latent features of the first training data; a decoder connected to the encoder and configured to reconstruct the masked divided data of the first training data based on the extracted latent features; and a representor connected to the encoder and configured to obtain representations for the contrastive learning based on the extracted latent features.

To achieve the aforementioned objects, a computing device for obtaining a neural network model for predicting a disease based on time-series data, comprises: a processor comprising at least one core; and a memory storing program code executable by the processor. The processor is configured to: obtain a plurality of pieces of first divided data by dividing first time-series data; obtain first training data corresponding to the first time-series data by masking at least one of the obtained plurality of pieces of first divided data; and train a first neural network model through self-supervised learning based on the first training data.

To achieve the aforementioned objects, a computer program stored on a computer-readable storage medium, when executed by one or more processors, causes operations for obtaining a neural network model for predicting a disease based on time-series data to be performed. The operations comprise: obtaining a plurality of pieces of first divided data by dividing first time-series data; obtaining first training data corresponding to the first time-series data by masking at least one of the obtained plurality of pieces of first divided data; and training a first neural network model through self-supervised learning based on the first training data.

### ADVANTAGEOUS EFFECTS

According to an embodiment of the present disclosure, the method for obtaining a neural network model for predicting a disease based on ECG data combines generative learning and contrastive learning using ECG data, thereby enabling the neural network model to effectively learn the latent features of ECG signals even in situations where labels are insufficient or data is limited. As a result, it is possible to overcome the limitations of training data while increasing the accuracy and reliability of heart disease prediction, and significantly improve efficiency and usability in the medical diagnosis field.

### BRIEF DESCRIPTION OF THE DRAWINGS

FIG. 1 is a block diagram of a computing device according to an embodiment of the present disclosure.
FIG. 2 is a flowchart of a method for obtaining a neural network model for predicting a disease based on ECG data according to an embodiment of the present disclosure.
FIG. 3 is an exemplary diagram of a method for obtaining a neural network model for predicting a disease based on ECG data according to an embodiment of the present disclosure.
FIG. 4 is a flowchart illustrating a method for training a first neural network model through generative learning and contrastive learning based on self-supervised learning according to an embodiment of the present disclosure.
FIG. 5 is a flowchart illustrating a method for training a second neural network model through generative learning based on labeled ECG data according to an embodiment of the present disclosure.
FIG. 6 is an exemplary diagram illustrating a method for training a second neural network model through supervised learning based on labeled ECG data according to an embodiment of the present disclosure.
FIG. 7 is an exemplary diagram of a method for generating a training dataset for contrastive learning using ECG data obtained from different subjects according to an embodiment of the present disclosure.
FIG. 8 is an exemplary diagram of a method for generating a training dataset for contrastive learning using a plurality of pieces of ECG data obtained from a single subject according to an embodiment of the present disclosure.
FIG. 9 is an exemplary diagram of a method for generating a training dataset for contrastive learning using a plurality of pieces of target ECG data obtained from a single subject according to an embodiment of the present disclosure.
FIG. 10 is an exemplary diagram of a method for generating a training dataset for contrastive learning using augmented data obtained by adjusting a plurality of pieces of ECG data obtained from a single subject according to an embodiment of the present disclosure.

### DETAILED DESCRIPTION OF THE EMBODIMENTS

Hereinafter, the present disclosure describes embodiments in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and is not to be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" is to be understood to mean any of the natural inclusive permutations. For example, if "X uses A or B" is used in the present disclosure, unless otherwise specified or clear from the context, it may be interpreted as any of the cases where X uses A, X uses B, or X uses both A and B.

The term "and/or" as used in the present disclosure is to be understood to refer to and include all possible combinations of one or more of the associated listed concepts.

The terms "comprise" and/or "comprising" as used in the present disclosure are to be understood to mean the presence of a specific feature and/or component. However, the terms "comprise" and/or "comprising" are to be understood as not precluding the presence or addition of one or more other features, other components, and/or combinations thereof.

In the present disclosure, unless otherwise specified or clear from the context to indicate a singular form, generally the singular is to be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "obtain" as used in the present disclosure may be understood as not only receiving data via a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing device or a set thereof, an application program that performs a specific function of software, a processing procedure implemented by software execution, or a set of instructions for program execution. Furthermore, in a broad sense, a "module" or a "unit" may refer to a computing device itself that constitutes a system, or an application executed on the computing device. However, the above-mentioned concepts are only examples, and the concepts of "module" or "unit" may be variously defined within the scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have problem-solving ability by optimizing parameters that connect nodes or neurons through learning. A neural network "model" may include a single neural network or a set of neural networks in which a plurality of neural networks are combined.

The term "data" as used in the present disclosure may include "images", signals, and the like. The term "image" as used in the present disclosure may refer to multi-dimensional data composed of discrete image elements. In other words, an "image" may be understood as a term referring to a digital representation of an object that can be seen by the human eye. For example, an "image" may refer to multi-dimensional data composed of elements corresponding to pixels in a 2D image. An "image" may refer to multi-dimensional data composed of elements corresponding to voxels in a 3D image.

The foregoing description of the terms is for the purpose of aiding the understanding of the present disclosure. Therefore, it is to be noted that if the foregoing terms are not explicitly stated as limiting the content of the present disclosure, they are not used in a sense that limits the technical spirit of the content of the present disclosure.

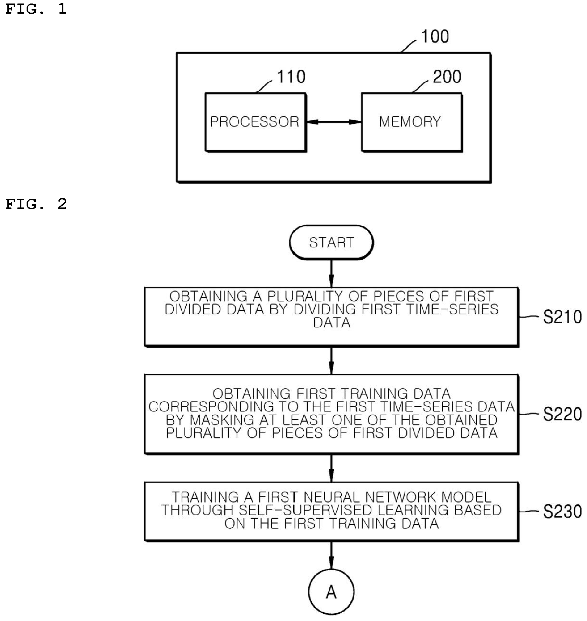

FIG. 1 is a block diagram of a computing device 100 according to an embodiment of the present disclosure.

Referring to FIG. 1, a computing device 100 comprises a processor 110 comprising at least one core (hereinafter "processor 110") and a memory 120.

However, FIG. 1 is only an example, and thus the computing device 100 may further comprise other components for implementing a computing environment. In addition, only some of the disclosed components may be included in the computing device 100.

The processor 110 according to an embodiment of the present disclosure may be understood as a component unit including hardware and/or software for performing computing operations. For example, the processor 110 may execute a computer program and perform data processing for machine learning. The processor 110 may process computational operations such as feature extraction for machine learning and error calculation based on backpropagation. The processor 110 for performing such data processing may include a central processing unit (CPU), a general-purpose graphics processing unit (GPGPU), a tensor processing unit (TPU), an application-specific integrated circuit (ASIC), or a field-programmable gate array (FPGA). The types of the processor 110 described above are only examples, and the type of the processor 110 may be variously configured within the scope understandable by a person of ordinary skill in the art based on the content of the present disclosure.

The computing device 100 may obtain training data required for training a neural network model from a plurality of subjects (e.g., patients). Specifically, the computing device 100 may configure training data with time-series data obtained from each subject. The time-series data may be data recorded sequentially over time, which is measured and obtained from a subject. The time-series data may include electrocardiography (ECG) data, photoplethysmography (PPG) data, electroencephalogram (EEG) data, electromyogram (EMG) data, and the like. However, hereinafter, for convenience of description of the present disclosure, the time-series data is assumed to be ECG data and described.

The processor 110 is electrically connected to other components of the computing device 100 (e.g., the memory 120) and controls the overall operation of the computing device 100.

A memory 120 according to an embodiment of the present disclosure may be understood as a constituent unit including hardware and/or software for storing and managing data processed in a computing device 100. That is, the memory 120 may store any form of data generated or determined by the processor 110 and any form of data received by the network unit of the computing device 100. For example, the memory 120 may include at least one storage medium of a flash memory type 120, a hard disk type, a multimedia card micro type, a card type memory 120, a random access memory (RAM), a static random access memory (SRAM), a read-only memory (ROM), an electrically erasable programmable read-only memory (EEPROM), a programmable read-only memory (PROM), a magnetic memory 120, a magnetic disk, or an optical disk. In addition, the memory 120 may include a database system that controls and manages data in a predetermined system. The foregoing types of the memory 120 are only one example, and the type of the memory 120 may be variously configured within a scope understandable by a person of ordinary skill in the art based on the content of the present disclosure.

The memory 120 may structure, organize, and manage data necessary for the processor 110 to perform operations, combinations thereof, and program code executable by the processor 110. For example, the memory 120 may store a training dataset used for training a neural network model and the neural network model. In addition, the memory 120 may store program code for training a neural network model through self-supervised learning, supervised learning, generative learning, or contrastive learning based on the training dataset, or for operating a trained neural network model, and processed data generated as the program code is executed.

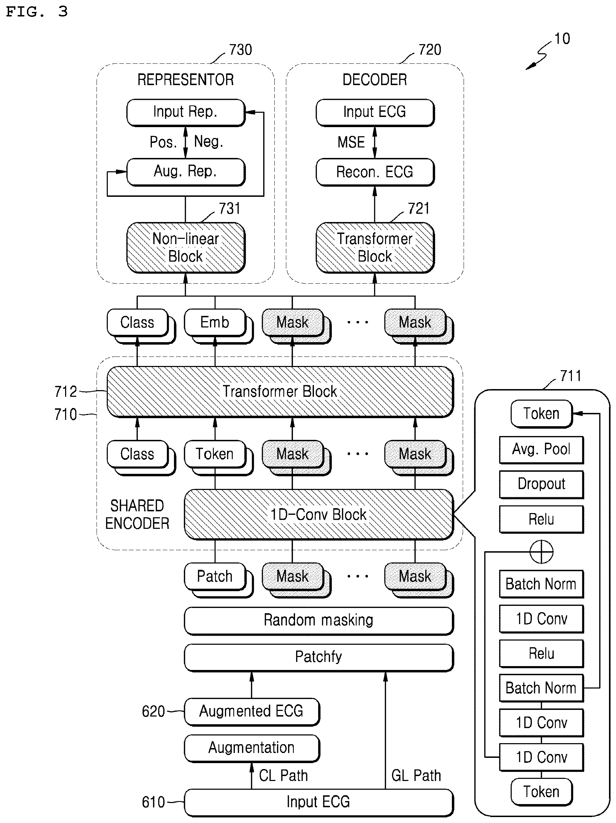

FIG. 2 is a flowchart of a method for obtaining a neural network model for predicting a disease based on ECG data according to an embodiment of the present disclosure. FIG. 3 is an exemplary diagram of a method for obtaining a neural network model for predicting a disease based on ECG data according to an embodiment of the present disclosure.

According to an embodiment of the present disclosure, the processor 110 obtains a plurality of pieces of first divided data by dividing first ECG data 610 (S310). The processor 110 may obtain the first ECG data 610 from other external electronic devices through a communication interface or obtain the first ECG data 610 stored in the memory 120. The first ECG data 610 comprises a plurality of pieces of ECG data obtained from a plurality of different patients. Therefore, the first ECG data 610 may also be referred to as a first training dataset. In particular, the first ECG data 610 may be ECG data to which labels, such as specific class information, are not assigned.

When the first ECG data 610 is obtained, the processor 110 may obtain a plurality of pieces of first divided data by dividing the first ECG data 610. Specifically, the processor 110 may divide each of the plurality of pieces of ECG data included in the first ECG data 610 into units of a preset size. In this process, the processor 110 may divide and process the first ECG data 610 into consecutive patch units along a time axis. For example, the processor 110 may obtain 10 patches by dividing the first ECG data 610 of 10-second length, obtained at a sample rate of 250 Hz, into 1-second units (250 points each) . Meanwhile, the processor 110 may set a patch size to comprise a PQRST cycle of an ECG signal corresponding to the ECG data, or may divide the patches so that a certain portion overlaps between adjacent patches. In this way, the processor 110 may obtain a plurality of pieces of first ECG data 610 corresponding to each of the pieces of ECG data included in the first ECG data 610.

Thereafter, the processor 110 may obtain first training data corresponding to the ECG data by masking at least one of the obtained plurality of pieces of first divided data (S320).

Specifically, the processor 110 may randomly select some of the plurality of pieces of first divided data (i.e., the plurality of patches) or select the pieces of first divided data at a certain ratio and mask them. In this case, the processor 110 may perform masking on the first divided data in a manner that covers or invalidates the entirety or a certain portion of the selected first divided data. The masking may be set to a continuous interval or a discontinuous interval, and the processor 110 may apply a value of 0 to the set interval or add noise to mask the first divided data. Through this masking process, the processor 110 may provide input data in an incomplete form so that the neural network model can learn the latent features of the first ECG data 610. In addition, the processor 110 may additionally assign masked tokens indicating masked positions, and may maintain the order of the original data. The processor 110 may obtain, as the first training data, a plurality of pieces of first divided data including the masked first divided data. In this case, the first training data includes a plurality of pieces of masked first divided data corresponding to a plurality of pieces of ECG data included in the first ECG data 610.

When the first training data is obtained, the processor 110 trains a first neural network model 10 through self-supervised learning based on the first training data (S330). The processor 110 may train the first neural network model 10 by inputting the plurality of pieces of masked first divided data included in the first training data to the first neural network model 10. The processor 110 may train the first neural network model 10 through self-supervised learning based on the first training data obtained from the first ECG data 610 to which a label is not assigned. The processor 110 may use the unlabeled first ECG data 610 to train the first neural network model through self-supervised learning. As an example, the processor 110 may train the first neural network model 10 to extract latent features of the first ECG data 610, and to predict or reconstruct the masked first divided data among the plurality of pieces of first divided data corresponding to the first ECG data 610.

To this end, according to an embodiment of the present disclosure, the processor 110 may train the first neural network model 10 to reconstruct the masked first divided data through a generative learning method based on self-supervised learning. Specifically, the processor 110 may obtain first output data by inputting the first training data to the first neural network model 10 so that the masked divided data of the first training data is reconstructed. Then, the processor 110 may train the first neural network model 10 through generative learning based on the first output data and the first ECG data 610.

In this case, referring to FIG. 3, the first neural network model 10 according to an embodiment of the present disclosure may comprise an encoder 710 - decoder 720. The first neural network model 10 may extract latent feature information of the input first training data with the encoder 710 and generate latent representation tokens. As an example, the encoder 710 may also generate latent representation tokens for the masked first divided data based on the features of the masked first divided data and other adjacent first divided data included in the first training data.

The encoder 710 may comprise a 1D convolution block 711 and a transformer block 712. A 1D convolution block 711 may be included at the input stage of the encoder 710. The 1D convolution block 711 processes a plurality of pieces of first divided data included in the first training data along a time axis, and may learn local features of an ECG signal corresponding to the first ECG data 610. As a result, each piece of first divided data may be converted into a feature map in a compressed form through a filtering process. The feature map may be transmitted to the transformer block 712 included at the output stage of the encoder 710. In addition, a classification token may be transmitted together to the transformer block 712. The classification token input to the transformer block 712 may be set to a learnable initial value. The transformer block 712 may learn a temporal correlation and global patterns between the masked first divided data and other adjacent divided data based on a self-attention mechanism. As a result, the transformer block 712 may finally output latent representation tokens and a classification token that summarizes the global features of the first training data. At this time, the latent representation tokens may include a feature representation that can be used to reconstruct the masked data or for contrastive learning.

The latent representation tokens extracted through the encoder 710 are transmitted to the decoder 720, and the decoder 720 may reconstruct the masked first divided data and the remaining first divided data and output data corresponding to the first ECG data 610 (i.e., first output data). In addition, a masked token that can identify the position of the masked first divided data may be input to the decoder 720. A plurality of latent representation tokens (and masked tokens) received from the encoder 710 are processed through a transformer block 721 of the decoder 720, and the transformer block 721 may learn a temporal relationship between the input latent representation tokens and masked tokens using a self-attention mechanism.

Meanwhile, the first output data obtained from the decoder 720 may be in the same form as the first ECG data 610, that is, in the form of entire ECG data in which a plurality of pieces of divided data reconstructed through a plurality of latent representation tokens are merged. The processor 110 may calculate the difference between the first output data thus obtained and the first ECG data 610 corresponding to the original data as a reconstruction loss, and may improve the reconstruction performance of the masked ECG data by repeatedly training the first neural network model 10 to minimize the reconstruction loss. In this case, a loss function of the generative learning (hereinafter "first loss function") may be as in Equation 1 below. ${L}_{R} = \frac{1}{N} {\sum }_{i = 1}^{N} \left|{x}_{i}-\hat{{x}_{i}}\right|$

In Equation 1, xᵢ is the i-th value of the original first ECG data 610, $\hat{{x}_{i}}$ is the i-th value of the reconstructed first output data, and N is the total number of points of the first ECG data 610.

According to an embodiment of the present disclosure, the processor 110 may train the first neural network model 10 through contrastive learning based on self-supervised learning based on the first training data in step S330. In particular, the processor 110 may train the first neural network model 10 through either generative learning or contrastive learning, or may perform them simultaneously in parallel.

Referring to FIG. 3, the first neural network model 10 according to an embodiment of the present disclosure may comprise a representor 730 connected to the encoder 710 to obtain representations for the contrastive learning based on the extracted latent features. Specifically, the representor 730 may receive the latent representation tokens (and classification tokens) transmitted from the encoder 710 and generate an embedding vector for performing contrastive learning. The representor 730 may be configured to generate an expression vector that can learn the similarity between a plurality of pieces of ECG data by utilizing both the features of the masked first divided data and the unmasked first divided data. Meanwhile, the representor 730 may comprise a non-linear block 731. The non-linear block 731 may suppress unnecessary features and emphasize core features by non-linearly transforming the latent representation tokens and classification tokens generated through the encoder 710.

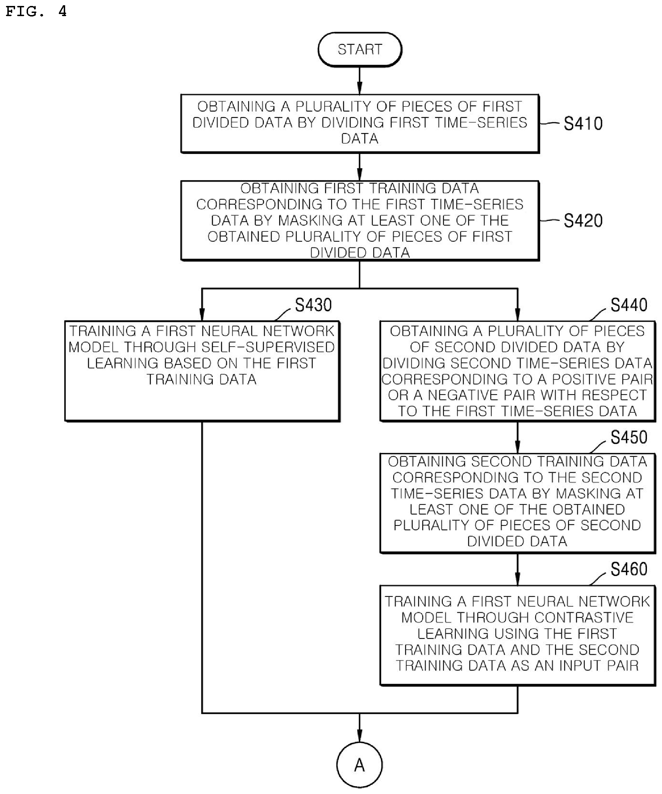

FIG. 4 is a flowchart illustrating a method for training a first neural network model 10 through generative learning and contrastive learning based on self-supervised learning according to an embodiment of the present disclosure. Steps S410 and S420 shown in FIG. 4 correspond to steps S210 and S220 shown in FIG. 2, and step S430 corresponds to the above-described generative learning, so a detailed description thereof is omitted.

The processor 110 may obtain a plurality of pieces of second divided data by dividing second ECG data 620 corresponding to a positive pair or a negative pair with respect to the first ECG data 610 (S440).

Contrastive learning is one of the self-supervised learning methods, a learning method that optimizes a neural network model to place similar data close to each other in an embedding space and to place different data far from each other by learning the relative representation between different data.

In the case of contrastive learning, for a plurality of training data constituting a positive pair, the similarity is maximized, and for a plurality of training data constituting a negative pair, the difference is maximized. Therefore, the processor 110 may obtain second training data that constitutes training data for a positive pair and training data for a negative pair with respect to each of the pieces of ECG data included in the first ECG data 610. The processor 110 may match at least one piece of ECG data included in the second training data constituting the positive pair to a specific piece of ECG data included in the first ECG data 610, and match at least one piece of ECG data included in the second training data constituting the negative pair with respect to the specific piece of ECG data. Thereafter, the processor 110 may train the first neural network model 10 to extract embedding vectors corresponding to a plurality of training data so that they are located closely in an embedding space for a plurality of training data constituting a positive pair, and to extract embedding vectors (or latent representation tokens) corresponding to a plurality of training data so that they are located far apart in an embedding space for a plurality of training data constituting a negative pair.

According to an embodiment of the present disclosure, when the first ECG data 610 and the second ECG data 620 are obtained from a single subject (e.g., a patient), the processor 110 may set the second ECG data 620 to correspond to a positive pair, and when the first ECG data 610 and the second ECG data 620 are obtained from different subjects, the processor 110 may set the second ECG data 620 to correspond to a negative pair.

Alternatively, the processor 110 may obtain augmented data corresponding to the first ECG data 610 by adjusting the first ECG data 610, and may set the obtained augmented data to correspond to a positive pair with respect to the first ECG data 610.

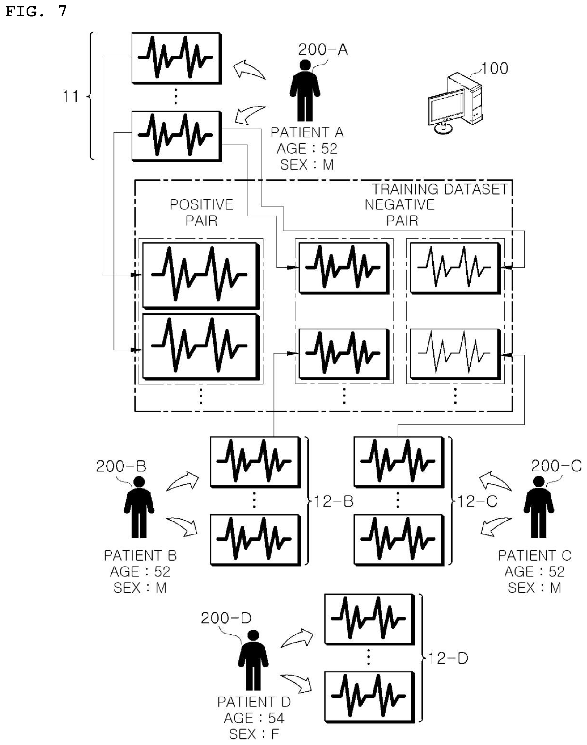

A method for configuring training data for a positive pair and training data for a negative pair is described in detail in FIGS. 7 to 9.

Meanwhile, when the second ECG data 620 corresponding to a positive pair or a negative pair with respect to the first ECG data 610 is obtained, the processor 110 may obtain a plurality of pieces of second divided data by dividing the second ECG data 620. The second ECG data 620 may be data comprising a plurality of pieces of ECG data that constitute a positive pair or a negative pair with respect to a plurality of pieces of ECG data included in the first ECG data 610. Therefore, the second ECG data 620 may also be referred to as a second training dataset. The second ECG data 620 may be divided into the same size and number as the first ECG data 610. Since the method of dividing the second ECG data 620 is the same as the method of dividing the first ECG data 610, a detailed description thereof is omitted.

Thereafter, the processor 110 may obtain second training data corresponding to the second ECG data 620 by masking at least one of the obtained plurality of pieces of second divided data (S450). The portion of the plurality of second divided data that is masked may be the same as the portion of the plurality of first divided data that is masked. For example, a portion of the plurality of second divided data may be masked at the same temporal position and with the same masking ratio as the masked first divided data. Since the method of masking the plurality of second divided data is the same as the method of masking the plurality of first divided data, a detailed description thereof is omitted.

The processor 110 may obtain, as the second training data, a plurality of pieces of second divided data including the masked second divided data. The second training data includes a plurality of pieces of masked second divided data corresponding to a plurality of pieces of ECG data included in the second ECG data 620. The processor 110 may train the first neural network model 10 through contrastive learning using the first training data and the second training data as an input pair (S460). The processor 110 may perform contrastive learning by inputting, to the first neural network model 10, the first training data and the second training data matched as a negative pair, or the first training data and the second training data matched as a positive pair (more specifically, the first training data and the second training data respectively obtained from a piece of ECG data included in the first ECG data 610 and a piece of second ECG data included in the second ECG data 620, which are matched as a negative pair or a positive pair).

Specifically, the processor 110 may obtain latent representation tokens corresponding to the first training data and the second training data respectively, by inputting the first training data and the second training data as an input pair to the encoder 710 of the first neural network model 10. Then, the processor 110 may generate an embedding vector by inputting the latent representation tokens corresponding to the first training data and the second training data respectively, which are output from the encoder 710, to the representor 730. The representor 730 refines the information of the latent representation tokens and may calculate an expression vector optimized for contrastive learning based on the learned features. For example, the representor 730 may remove unnecessary features and extract only core features in the process of generating an expression vector optimized for contrastive learning based on the latent representation tokens. The expression vector obtained from the representor 730 may be a criterion for comparing the similarity or difference between data. The processor 110 may, in the contrastive learning process, train the first neural network model 10 to increase the similarity between expression vectors for a positive pair and to decrease the similarity between expression vectors for a negative pair. The similarity may be calculated mainly based on cosine similarity. Meanwhile, the processor 110 may perform optimization through a contrastive loss. In this case, a loss function of the contrastive learning (hereinafter "second loss function") may be as in Equation 2 below. ${L}_{C} = - log \frac{exp \left(sim\left({z}_{i}, {z}_{j}\right)/τ\right)}{{\sum }_{k = 1}^{2 N} 1 \left[k\neq i\right] exp \left(sim\left({z}_{i}, {z}_{k}\right)/τ\right.}$

In Equation 2, sim(zi, zj) is the cosine similarity between the two expression vectors of a positive pair, sim(zi, zk) is the cosine similarity between the two expression vectors of a negative pair, τ is a temperature scaling parameter, and N is the total number of input data.

In this case, the processor 110 may apply, to the first neural network model 10, a combination of a first loss function corresponding to the generative learning and a second loss function corresponding to the contrastive learning. Specifically, the processor 110 may set a combined loss function by combining the first loss function and the second loss function as in Equation 3 below. ${L}_{total} = {L}_{R} + λ {L}_{c}$

λ may be a parameter that adjusts the balance between the generative learning and the contrastive learning, as a weight of the contrastive loss function.

In conclusion, the processor 110 may apply a combination of the first loss function and the second loss function so that the generative learning and the contrastive learning can be performed simultaneously on the first neural network model 10, thereby improving the reconstruction performance of the first neural network model 10 for the masked first ECG data 610, and may train the first neural network model 10 to sophisticatedly distinguish the latent feature representations between a positive pair and a negative pair.

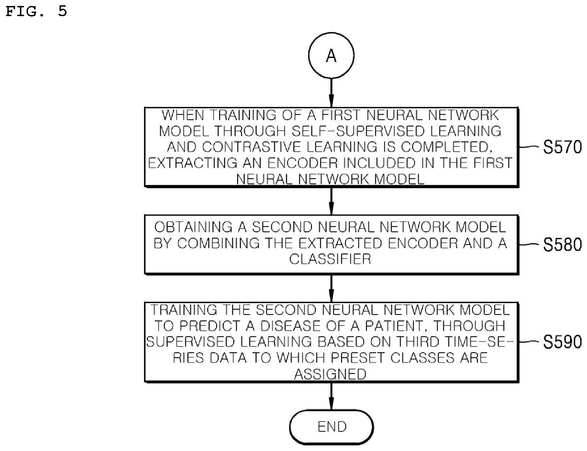

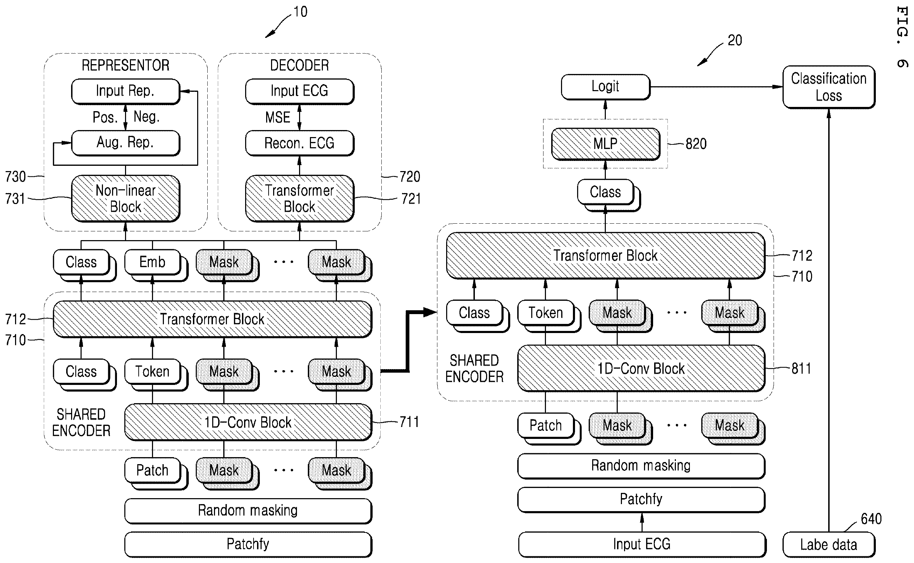

FIG. 5 is a flowchart illustrating a method for training a second neural network model 20 through supervised learning based on labeled ECG data according to an embodiment of the present disclosure. FIG. 6 is an exemplary diagram illustrating a method for training the second neural network model 20 through supervised learning based on labeled ECG data according to an embodiment of the present disclosure.

Steps S510 to S560 shown in FIG. 5 may correspond to steps S410 to S460 shown in FIG. 4, respectively. Therefore, a detailed description thereof is omitted.

Referring to FIG. 5, the processor 110 may extract an encoder 710 included in the first neural network model 10 when the training of the first neural network model 10 through generative learning or contrastive learning is completed (S570). The encoder 710 may be trained to perform the role of converting input ECG data into latent representation tokens and classification tokens, having learned both the local features and global patterns of ECG signals based on the first ECG data 610 (and the second ECG data 620).

Thereafter, the processor 110 may obtain a second neural network model 20 by combining the extracted encoder 710 and a classifier 820 (S580). Referring to FIG. 6, the processor 110 extracts the encoder 710 from the first neural network model 10, for which the generative learning and the contrastive learning have been completed, and obtains the second neural network model 20 by combining the extracted encoder 710 and the classifier 820. The classifier 820 may have a multi-layer perceptron (MLP) structure, and may be designed to output a class probability value of the ECG data input to the encoder 710 based on the classification token output from the encoder 710.

The processor 110 fine-tunes the second neural network model 20 to predict a disease of a patient, through supervised learning based on third ECG data 630 to which preset classes are assigned (S590). The third ECG data 630 comprises a plurality of pieces of ECG data obtained from a plurality of different patients. Therefore, the third ECG data 630 may also be referred to as a third training dataset. In particular, the third ECG data 630 may be ECG data to which labels, such as specific class information, are assigned. The specific class is information indicating the health status or disease of a patient, and may comprise disease types such as normal, myocardial infarction, arrhythmia, and hypertrophy.

The processor 110 may divide the third ECG data 630 into patch units and input them to the encoder 710 of the second neural network model 20 to generate a classification token. The third ECG data 630 may be divided into patch units of the same size and number as the first ECG data 610 used to train the encoder 710. The classification token extracted through the encoder 710 is transmitted to the classifier 820 (MLP), and the classifier 820 outputs a probability value for each class based on the input classification token. The processor 110 calculates a classification loss between the predicted class probability value and the actual label 640 assigned to the third ECG data 630, and may repeatedly adjust and optimize the parameters of the second neural network model 20 to minimize it. As a result, the processor 110 may fine-tune the second neural network model 20 to predict a disease of a patient corresponding to a specific class assigned to the third ECG data 630.

According to an embodiment of the present disclosure, when the processor 110 obtains a plurality of pieces of ECG data corresponding to a specific subject, the processor 110 may selectively combine two of the plurality of pieces of ECG data obtained from the specific subject to obtain one or more positive pairs. Thereafter, the processor 110 may selectively combine one from a plurality of pieces of ECG data obtained from the specific subject and one from a plurality of pieces of ECG data obtained from another subject to obtain one or more negative pairs.

That is, the processor 110 may select two pieces of ECG data from a plurality of pieces of ECG data obtained from a subject, and obtain the selected two pieces of ECG data as a positive pair of the first training data and the second training data. In particular, the two pieces of ECG data obtained as a positive pair from the same subject may be selected regardless of the time or order in which the ECG data were obtained. In addition, the processor 110 may select one piece of ECG data from a plurality of pieces of ECG data obtained from a specific subject, and select one piece of ECG data from a plurality of pieces of ECG data obtained from another subject, and obtain them as a negative pair of the first training data and the second training data. The obtained negative pair may also be a negative pair of the training data for another subject. That is, a negative pair obtained for a specific subject may be shared with another subject corresponding to the ECG data included in the negative pair.

FIG. 7 is an exemplary diagram of a method for generating a training dataset for contrastive learning using ECG data obtained from different subjects according to an embodiment of the present disclosure.

Referring to FIG. 7, the processor 110 may obtain a plurality of pieces of ECG data (11, 12-B, 12-C, and 12-D) from patient A (200-A), patient B (200-B), patient C (200-C), and patient D (200-D), respectively. In obtaining a training dataset for contrastive learning for patient A (200-A), the processor 110 may select two from a plurality of pieces of ECG data 11 obtained from patient A (200-A) to obtain a positive pair of the training dataset. In addition, the processor 110 may combine one of a plurality of pieces of ECG data 11 obtained from patient A (200-A) and one of a plurality of pieces of ECG data (12-B and 12-C) obtained from patient B (200-B) or patient C (200-C) to obtain a negative pair included in the training dataset for patient A (200-A). This applies equally to patient B (200-B) and patient C (200-C).

In this case, according to an embodiment of the present disclosure, another subject corresponding to another piece of ECG data that constitutes a negative pair with the ECG data of a specific subject may have similar biological information. The biological information may include at least one of a subject's age, sex, height, and weight. To this end, the processor 110 may identify the biological information of the subject and another subject, and obtain ECG data for obtaining a negative pair from another subject having biological information similar to the biological information of the subject. The processor 110 may determine that a plurality of subjects in which at least one of age, sex, height, and weight matches have similar biological information.

Referring again to FIG. 7, the processor 110 may identify that patient A (200-A), patient B (200-B), and patient C (200-C) have the same age and sex, while patient D (200-D) has a different age and sex. Therefore, in obtaining a negative pair of the training dataset of patient A (200-A), the processor 110 may not use the ECG data (12-D) of patient D (200-D).

To this end, the processor 110 may calculate the similarity of biological information between the subject and another subject. For example, the processor 110 may extract a plurality of vectors corresponding to the biological information for each subject, and may calculate the similarity of the biological information based on the distance between the extracted vectors. Thereafter, the processor 110 may combine the ECG data of a plurality of subjects whose similarity is equal to or more than a preset value to obtain a negative pair of the training dataset.

Meanwhile, according to an embodiment of the present disclosure, the processor 110 may obtain a positive pair and a negative pair of a training dataset from a plurality of pieces of ECG data obtained from a single subject. In particular, the processor 110 may obtain a positive pair and a negative pair of a training dataset based on a plurality of pieces of ECG data obtained from a specific subject when the number of positive pairs obtained is less than a preset number, or when the number of negative pairs obtained based on ECG data obtained from another subject is less than a preset number. Hereinafter, an embodiment of the present disclosure related thereto is described.

According to an embodiment of the present disclosure, the processor 110 may obtain a plurality of pieces of ECG data corresponding to a subject over multiple sessions and then set reference data among the plurality of pieces of ECG data. The reference data may be ECG data that serves as a criterion for selecting ECG data to be included in a negative pair (and a positive pair) among a plurality of pieces of ECG data obtained from a single subject.

According to an embodiment of the present disclosure, the processor 110 may set the ECG data first obtained from a subject among a plurality of pieces of ECG data obtained from the subject as the reference data.

In this case, the processor 110 may obtain one or more positive pairs by selectively combining two pieces of data from the reference data and a plurality of pieces of ECG data obtained within a preset time from the time point when the reference data was obtained, and may obtain one or more negative pairs by selectively combining the reference data and one of a plurality of pieces of ECG data obtained after a preset time from the time point when the reference data was obtained.

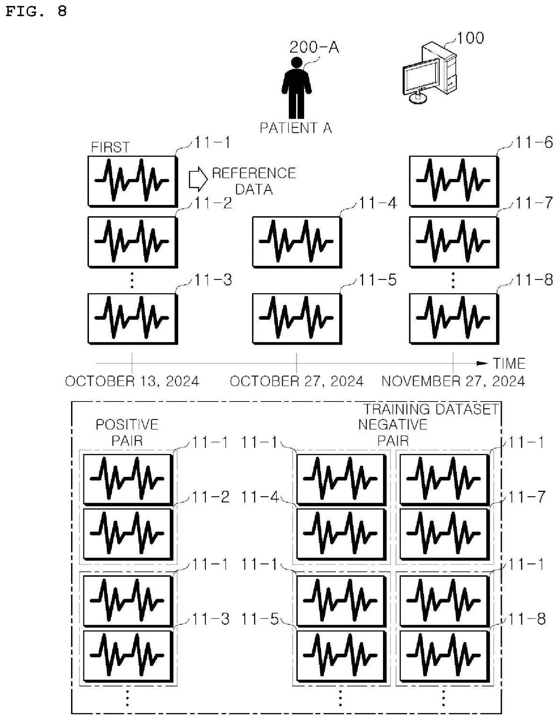

FIG. 8 is an exemplary diagram of a method for generating a training dataset for contrastive learning using a plurality of pieces of ECG data obtained from a single subject according to an embodiment of the present disclosure.

Specifically, the processor 110 may identify the time point when the ECG data set as the reference data for the subject was obtained. The processor 110 may select a plurality of pieces of ECG data obtained within a preset time from the time point when the reference data was obtained. Thereafter, the processor 110 may selectively combine two pieces of data from the reference data and the selected plurality of pieces of ECG data to obtain a positive pair. As an example, referring to FIG. 8, the processor 110 sets the ECG data (i.e., the ECG data obtained on October 13, 2024) (11-1) first obtained among a plurality of pieces of ECG data (11-1 to 11-8, hereinafter "11") as the reference data, and in order to obtain a positive pair, may select a plurality of pieces of ECG data (11-1 to 11-3) obtained on the same date as the date when the reference data was obtained (October 13, 2024). A plurality of pieces of ECG data (11-1 to 11-3) on the same date may be obtained by patient A (200-A) visiting a hospital and measuring an ECG signal over multiple sessions. Then, the processor 110 may select two from the pieces of ECG data (11-1 to 11-3) obtained on the same date as the date when the reference data was obtained by the patient visiting the hospital, and obtain a positive pair.

In addition, the processor 110 may select a plurality of pieces of ECG data obtained after a preset time from the time point when the reference data was obtained. Thereafter, the processor 110 may combine the reference data and one piece of ECG data selected from among the selected plurality of pieces of ECG data to obtain a negative pair. As an example, referring again to FIG. 8, in order to obtain a negative pair, the processor 110 may select a plurality of pieces of ECG data (11-4 to 11-8) obtained on different dates (October 27, 2024 and November 27, 2024) from the date when the reference data was obtained (October 13, 2024). That is, the processor 110 may select the ECG data (11-4 to 11-8) obtained as the patient visits the hospital again after the reference data was obtained, and obtain a negative pair.

Meanwhile, the processor 110 may obtain a plurality of pieces of ECG data by dividing the ECG data obtained from a subject. In this case, the ECG data to be divided may correspond to an ECG signal longer than the ECG signal corresponding to the ECG data. Hereinafter, for convenience of description of the present disclosure, the ECG data corresponding to a long-time measured ECG signal to be divided is referred to as target ECG data.

The processor 110 may obtain a plurality of pieces of ECG data by dividing the target ECG data at a preset time interval. For example, if the ECG data is obtained by sampling an ECG signal of 10-second length, the target ECG data may be obtained by sampling an ECG signal of a length exceeding 10 seconds. That is, ECG data corresponding to an ECG signal of 10-second length may be obtained by dividing an ECG signal of a length exceeding 10 seconds by 10 seconds. Alternatively, the processor 110 may obtain a plurality of pieces of ECG data corresponding to an ECG signal of 10-second length by dividing target ECG data corresponding to an ECG signal of a length exceeding 10 seconds.

Meanwhile, the processor 110 may obtain a plurality of pieces of ECG data corresponding to each target ECG data by dividing each of a plurality of pieces of target ECG data.

In this case, according to an embodiment of the present disclosure, the processor 110 may set reference data among a plurality of pieces of ECG data. As described above, the processor 110 may set the ECG data first obtained among a plurality of pieces of ECG data as the reference data. Alternatively, the processor 110 may identify the target ECG data with the largest number of divided pieces of ECG data among a plurality of pieces of target ECG data, and may set reference data from a plurality of pieces of ECG data corresponding to the identified target ECG data. In addition, among a plurality of pieces of ECG data corresponding to the identified target ECG data, the ECG data located in the central part of the target ECG data or the ECG data first obtained may be set as the reference data.

Thereafter, the processor 110 may obtain one or more positive pairs by selectively combining two pieces of data from the reference data and a plurality of pieces of ECG data obtained by dividing from the same target ECG data as the reference data, and may obtain one or more negative pairs by selectively combining the reference data and one of a plurality of pieces of ECG data obtained by dividing from a different target ECG data than the reference data.

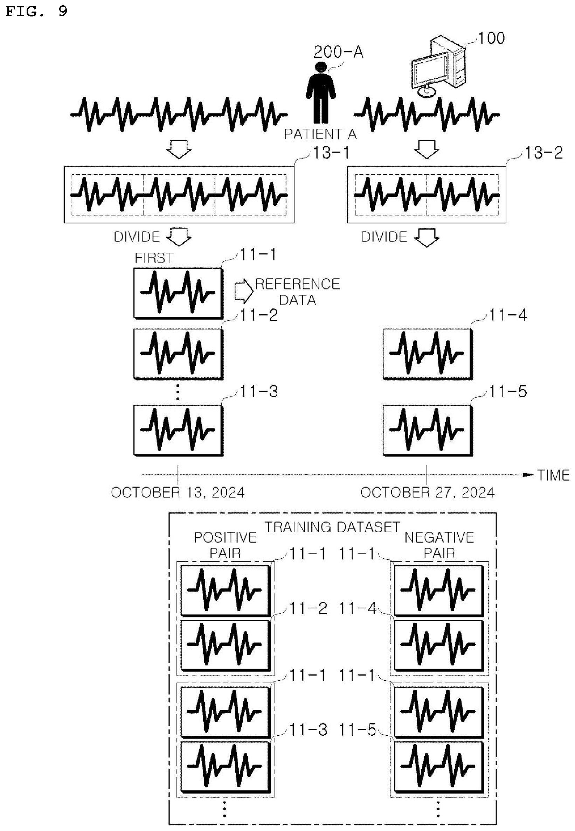

FIG. 9 is an exemplary diagram of a method for generating a training dataset for contrastive learning using a plurality of pieces of target ECG data obtained from a single subject according to an embodiment of the present disclosure.

Referring to FIG. 9, the processor 110 may obtain a plurality of pieces of ECG data (11-1 to 11-5) by dividing each of target ECG data (13-1) obtained on October 13, 2024 and target ECG data (13-2) obtained on October 27, 2024. In this case, the processor 110 sets the ECG data (11-1) first obtained among a plurality of pieces of ECG data (11-1 to 11-3) obtained by dividing the target ECG data (13-1) obtained on October 13, 2024 as the reference data, and may obtain a positive pair of the training dataset by combining the reference data and the remaining ECG data (11-2 and 11-3) obtained from the same target ECG data (13-1) as the reference data. In addition, the processor 110 may obtain a negative pair of the training dataset by combining the reference data (11-1) and one piece of data selected from the remaining ECG data (11-4 and 11-5) obtained from a different target ECG data (the target ECG data (13-2) obtained on October 27, 2024) than the reference data.

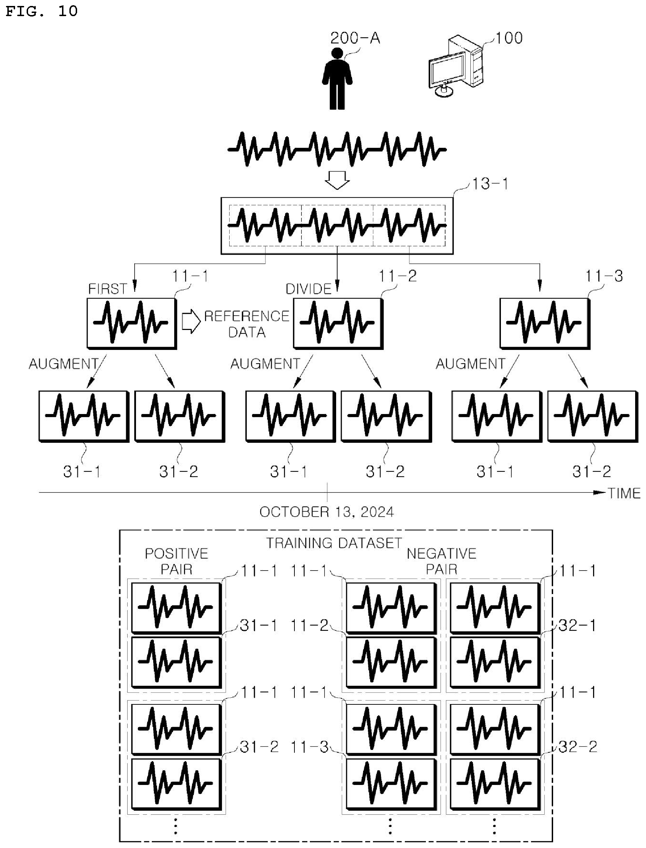

FIG. 10 is an exemplary diagram of a method for generating a training dataset for contrastive learning using augmented data obtained by adjusting a plurality of pieces of ECG data obtained from a single subject according to an embodiment of the present disclosure.

Alternatively, according to an embodiment of the present disclosure, the processor 110 may obtain a plurality of pieces of augmented data corresponding to the reference data (hereinafter "first augmented data") by adjusting the set reference data, and may obtain a plurality of pieces of augmented data corresponding to the remaining ECG data (hereinafter "second augmented data") by adjusting at least one remaining piece of ECG data other than the reference data. The processor 110 may obtain augmented data corresponding to each piece of ECG data by adjusting each piece of ECG data. For example, the processor 110 may adjust the ECG data by adding baseline noise or muscle artifacts to the ECG signal corresponding to the ECG data to fluctuate the baseline of the waveform of the ECG signal. This may also be performed by applying a baseline noise addition pattern or a muscle artifact addition pattern. Alternatively, the processor 110 may adjust the ECG data by adding white noise to the ECG data or by applying a partial zero padding method. In this way, the processor 110 may obtain augmented data corresponding to each piece of ECG data by adjusting each of a plurality of pieces of ECG data.

The processor 110 may obtain one or more positive pairs by selectively combining two pieces of data from the reference data and a plurality of pieces of first augmented data corresponding to the reference data, and may obtain one or more negative pairs by selectively combining the reference data and one of a plurality of pieces of second augmented data corresponding to a plurality of pieces of ECG data other than the reference data.

Referring to FIG. 10, the processor 110 may obtain a plurality of pieces of ECG data (11-1 to 11-3) by dividing target ECG data (13-1) obtained on October 13, 2024. In this case, the processor 110 sets the ECG data (11-1) first obtained among a plurality of pieces of ECG data (11-1 to 11-3) obtained by dividing the target ECG data (13-1) obtained on October 13, 2024 as the reference data, and obtains a plurality of pieces of first augmented data (31-1 and 31-2) by adjusting the reference data. Thereafter, the processor 110 may obtain a plurality of pieces of second augmented data (32-1 to 32-4) by adjusting the ECG data (11-2 and 11-3) other than the reference data. Thereafter, the processor 110 may obtain a positive pair of the training dataset by combining the reference data and one selected from a plurality of pieces of first augmented data (31-1 and 31-2), and may obtain a negative pair of the training dataset by combining the reference data and one selected from a plurality of pieces of second augmented data (32-1 to 32-4).

In this case, a first augmentation method for obtaining a plurality of pieces of first augmented data and a second augmentation method for obtaining a plurality of pieces of second augmented data may be different. For example, while the first augmentation method is a white noise addition method, the second augmentation method may be a partial zero padding method.

Meanwhile, the processor 110 may train the first neural network model 10 through contrastive learning based on a training dataset used for contrastive learning, which is composed of the generated first training data and second training dataset. That is, the processor 110 may train the first neural network model 10 through contrastive learning based on a training dataset comprising the obtained positive pairs and negative pairs. In particular, the processor 110 may train the first neural network model 10 through contrastive learning so that the distance between a plurality of embedding vectors corresponding to a plurality of pieces of ECG data included in a positive pair becomes closer in an embedding space, and the distance between a plurality of embedding vectors corresponding to a plurality of pieces of ECG data included in a negative pair becomes farther in an embedding space. To this end, the processor 110 may perform learning using a contrastive loss function or an InfoNCE loss function to minimize the distance between the embedding vectors of a positive pair and to maximize the distance between the embedding vectors of a negative pair.

The second neural network model 20 comprising the encoder 710 for which contrastive learning has been completed may be effectively utilized to analyze ECG data and predict the health status of a patient. The second neural network model 20 comprising the encoder 710, which has learned the similarity and difference of various ECG patterns through contrastive learning, may more accurately interpret the ECG data of a patient by forming an embedding space that distinguishes normal and abnormal patterns. Accordingly, patient-specific disease prediction and monitoring become possible, and the risk of heart disease may be predicted more quickly.

The second neural network model 20 comprising the encoder 710 for which contrastive learning has been completed may perform basic analysis of ECG patterns without new labeling through zero-shot learning, and may increase the learning and prediction accuracy for a specific disease with a small amount of data through few-shot learning. The second neural network model 20 comprising the encoder 710 for which contrastive learning has been completed may be extended to a model specialized for the diagnosis of a specific disease by performing fine-tuning with data related to the specific disease.

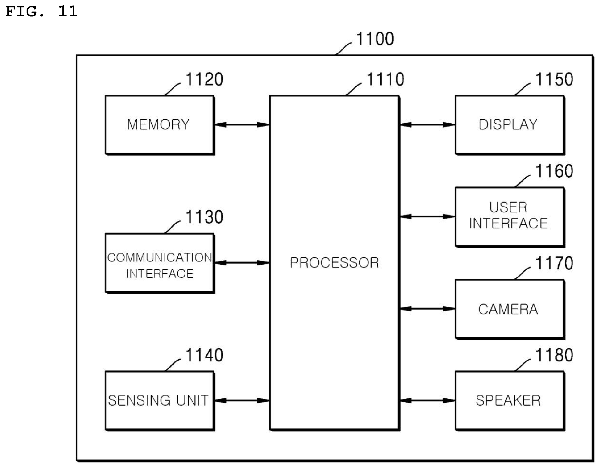

FIG. 11 is a block diagram of a computing device 1100 according to another embodiment of the present disclosure.

Referring to FIG. 11, a computing device 1100 according to an embodiment of the present disclosure comprises a processor 1110, a memory 1120, a communication interface 1130, a sensing unit 1140, a display 1150, a user interface 1160, a camera 1170, and a speaker 1180. Among the components shown in FIG. 10, the processor 1110 and the memory 1120 correspond to the configurations of the processor 110 and the memory 120 of the computing device 100 shown in FIG. 1, and therefore a detailed description thereof is omitted.

According to an embodiment of the present disclosure, the communication interface 1130 according to an embodiment of the present disclosure may be understood as a component unit that transmits and receives data through any form of known wired/wireless communication system. For example, the communication interface 1130 may perform data transmission and reception using a wired/wireless communication system such as a local area network (LAN), wideband code division multiple access (WCDMA), long term evolution (LTE), wireless broadband internet (WiBro), 5th generation mobile communication (5G), ultra wideband, ZigBee, radio frequency (RF) communication, wireless LAN, wireless fidelity (Wi-Fi), near field communication (NFC), or Bluetooth. The communication systems described above are only examples, and therefore, wired/wireless communication systems for data transmission and reception of the communication interface 1130 may be variously applied in addition to the examples described above.

The communication interface 1130 may receive data required for the processor 1110 to perform operations via wired/wireless communication with any system or any client. In addition, the communication interface 1130 may transmit data generated by the operations of the processor 1110 via wired/wireless communication with any system or any client. For example, the communication interface 1130 may receive medical data via communication with a database in a hospital environment, a cloud server that performs tasks such as standardization of medical data, or a computing device 1100. The communication interface 1130 may transmit output data of the second neural network model, and intermediate data and processed data derived from the operation process of the processor 1110, via communication with the aforementioned database, server, or computing device 1100. As an example, the processor 1110 may obtain biosignal data of a subject 1 and a plurality of pieces of ECG data of each subject from an external computing device (e.g., an external server device or an external biosignal measurement device) via the communication interface 1130.

The sensing unit 1140 may obtain biosignal data of a subject. As an example, the sensing unit 1140 may comprise a plurality of electrodes (e.g., 12 leads). In this case, the processor 1110 may obtain an ECG signal of a user as biosignal data via at least one electrode. In addition, the sensing unit 1140 may comprise an image sensor or an optical sensor. In this case, the processor 1110 may obtain a photoplethysmogram signal of a user as biosignal data via the image sensor (or the optical sensor).

The display 1150 may display various images. The images include both still images and moving images. The display 1150 may output guide information about activities generated based on a user's state. The display 1150 may be implemented as various types of displays such as a liquid crystal display panel (LCD), an organic light emitting diode (OLED), a liquid crystal on silicon (LCoS), and a digital light processing (DLP). In addition, the display 1150 may also comprise a driving circuit, a backlight unit, and the like, which can be implemented in a form such as an a-Si TFT, a low temperature poly silicon (LTPS) TFT, or an organic TFT (OTFT).

Meanwhile, the display 1150 may be combined with a touch panel to be implemented as a touch screen, and in this case, the display 1150 may perform the function of an input interface for receiving a user's touch input as well as an output interface for outputting an image via the touch screen. The display 1150 may display a determination result about the possibility of a heart disease predicted using the trained second neural network model, a treatment plan, and management information of the subject 1.

The user interface 1160 is a component used for the computing device 1100 to perform an interaction with a user, and may include at least one of a touch sensor, a motion sensor, a button, a jog dial, and a switch, but is not limited thereto. The processor 1110 may receive a user's biological information (e.g., occupation, age, sex) via the user interface 1160.

The camera 1170 captures an object around a user to obtain an image of the object. Specifically, the camera 1170 may obtain an image of food consumed by a user. In this case, the processor 1110 may identify the nutritional status of the user based on the user's state and the image of the food consumed by the user, and may provide recommended diet information related to a heart disease as guide information. To this end, the camera 1170 may be implemented as an imaging device having a CMOS structure (CIS, CMOS Image Sensor), or an imaging device having a CCD structure (Charge Coupled Device). However, it is not limited thereto, and the camera 1170 may be implemented as a camera module of various resolutions that can capture a subject. Meanwhile, the camera 1170 may be implemented as a depth camera (e.g., an IR depth camera), a stereo camera, or an RGB camera.

The speaker 1180 may output various audio data on which various processing operations, such as decoding, amplification, and noise filtering, have been performed by an audio processing unit (not shown). The speaker 1180 may output various alarm sounds or voice messages. According to an embodiment of the present disclosure, the processor 1110 may convert an electrical signal received from an external device into a user's voice and output it via the speaker 1180. As an example, the speaker 1180 may output a voice message that warns of a heart disease or suggests a diagnosis based on a determination result about the possibility of a heart disease identified through the second neural network model.

The various embodiments of the present disclosure described above may be combined with additional embodiments, and may be modified within the scope understandable by a person of ordinary skill in the art in light of the foregoing detailed description. The embodiments of the present disclosure are intended to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts are to be interpreted as being included in the scope of the present disclosure.

## Claims

1. A method for obtaining a neural network model for predicting a disease based on time-series data, performed by a computing device comprising at least one processor, the method comprising:
obtaining a plurality of pieces of first divided data by dividing first time-series data;
obtaining first training data corresponding to the first time-series data by masking at least one of the obtained plurality of pieces of first divided data; and
training a first neural network model through self-supervised learning based on the first training data.

2. The method of claim 1, wherein
the training of the first neural network model comprises:
training the first neural network model through generative learning or contrastive learning based on the first training data.

3. The method of claim 2, wherein
the training of the first neural network model through the generative learning or the contrastive learning comprises:
training the first neural network model through the generative learning based on the first time-series data and output data, the output data being obtained by inputting the first training data to the first neural network model so that the masked divided data of the first training data is reconstructed.

4. The method of claim 2, the method comprising:
obtaining a plurality of pieces of second divided data by dividing second time-series data corresponding to a positive pair or a negative pair with respect to the first time-series data; and
obtaining second training data corresponding to the second time-series data by masking at least one of the obtained plurality of pieces of second divided data,
wherein the training of the first neural network model through the generative learning or the contrastive learning comprises:
training the first neural network model through the contrastive learning using the first training data and the second training data as an input pair.

5. The method of claim 2, the method comprising:
extracting an encoder included in the first neural network model when the training of the first neural network model through the generative learning or the contrastive learning is completed;
obtaining a second neural network model by combining the extracted encoder and a classifier; and
fine-tuning the second neural network model to predict a disease of a patient, through supervised learning based on third time-series data to which preset classes are assigned.

6. The method of claim 4, wherein
the obtaining of the plurality of pieces of second divided data comprises:
setting the second time-series data to correspond to a positive pair when the first time-series data and the second time-series data are obtained from a single subject, and to a negative pair when the first time-series data and the second time-series data are obtained from different subjects.

7. The method of claim 4, wherein
the obtaining of the plurality of pieces of second divided data comprises:
setting augmented data corresponding to the first time-series data to correspond to a positive pair with respect to the first time-series data, the augmented data being obtained by adjusting the first time-series data.

8. The method of claim 2, wherein
the training of the first neural network model through the generative learning or the contrastive learning comprises:
applying, to the first neural network model, a combination of a first loss function corresponding to the generative learning and a second loss function corresponding to the contrastive learning.

9. The method of claim 2, wherein
the first neural network model comprises:
an encoder configured to extract latent features of the first training data;
a decoder connected to the encoder and configured to reconstruct the masked divided data of the first training data based on the extracted latent features; and
a representor connected to the encoder and configured to obtain representations for the contrastive learning based on the extracted latent features.

10. A computing device for obtaining a neural network model for predicting a disease based on time-series data, the computing device comprising:
a processor comprising at least one core; and
a memory storing program code executable by the processor,
wherein the processor is configured to:
obtain a plurality of pieces of first divided data by dividing first time-series data;
obtain first training data corresponding to the first time-series data by masking at least one of the obtained plurality of pieces of first divided data; and
train a first neural network model through self-supervised learning based on the first training data.

11. A computer program stored on a computer-readable storage medium, the computer program, when executed by one or more processors, causing operations for obtaining a neural network model for predicting a disease based on time-series data to be performed,
the operations comprising:
obtaining a plurality of pieces of first divided data by dividing first time-series data;
obtaining first training data corresponding to the first time-series data by masking at least one of the obtained plurality of pieces of first divided data; and
training a first neural network model through self-supervised learning based on the first training data.
